(19) Europäisches Patentamt
European Patent Office
Office européen des brevets

(11) Publication number: **0 291 269**
**A2**

(12) # EUROPEAN PATENT APPLICATION

(21) Application number: 88304209.5

(22) Date of filing: 10.05.88

(51) Int. Cl.4: C07D 333/72, C07D 333/54, C07D 209/08, A61K 31/38, A61K 31/40, A61K 31/64

The title of the invention has been amended (Guidelines for Examination in the EPO, A-III, 7.3).

(30) Priority: 12.05.87 US 49185

(43) Date of publication of application: 17.11.88 Bulletin 88/46

(84) Designated Contracting States: AT BE CH DE ES FR GB GR IT LI NL SE

(71) Applicant: **ELI LILLY AND COMPANY**
**Lilly Corporate Center**
**Indianapolis Indiana 46285(US)**

(72) Inventor: **Harper, Richard Waltz**
**4470 North Delaware Street**
**Indianapolis Indiana 46205(US)**
Inventor: **Howbert, James Jeffry**
**5720 North Ewing Street**
**Indianapolis Indiana 46220(US)**
Inventor: **Poore, Gerald Auston**
**1065 Old Moore Road**
**Martinsville Indiana 46151(US)**
Inventor: **Rieder, Brent Jeffrey**
**510 North State Street**
**Greenfield Indiana 46140(US)**
Inventor: **Tao, Eddie Vi-Ping**
**1211 Kirkham Lane**
**Indianapolis Indiana 46260(US)**

(74) Representative: **Tapping, Kenneth George et al**
**Erl Wood Manor**
**Windlesham Surrey, GU20 6PH(GB)**

(54) **Anti-tumour sulfonyl urea derivatives.**

(57) This invention provides certain sulfonamide derivatives, their pharmaceutical formulations, and their use in the treatment of susceptible neoplasms in mammals.

EP 0 291 269 A2

## ANTI-TUMOR METHOD AND COMPOUNDS

Despite the development of numerous chemical agents and sophisticated regimens of drug therapy, the ravages of cancer continue to extract an ever-increasing human toll of suffering and death. Although many advances have been made, especially in the area of combination drug therapy, the need for new and better methods of treating neoplasms and leukemias has not diminished. This is especially evident in the area of inoperable or metastatic solid tumors, such as various forms of lung cancer.

To be especially useful, new chemotherapeutic agents should have a wide spectrum of activity, a large therapeutic index, and be chemically stable and compatible with other agents. In addition, any new agents having oral activity would be especially useful so that initial treatment and subsequent maintenance therapy would be made easily and without inconvenience or pain to the patient.

We have discovered a series of sulfonylureas which are useful in the treatment of solid tumors. The compounds are orally active and relatively non-toxic providing an excellent therapeutic index.

This invention provides sulfonylurea derivatives of the formula

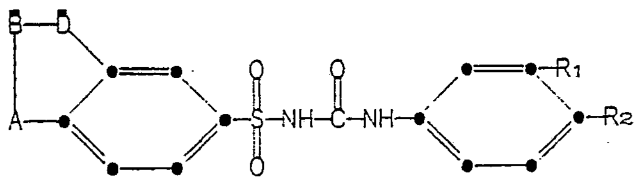

I

wherein A is $-S(O)_n-$, $-CH_2S(O)_n-$, -N(ethyl)-, or $-CH_2-$;
D is $-CH_2-$, $-S(O)_n-$, -NR-, or $-CH_2S(O)_n-$;
B is $-CH_2$, -O-, $-S(O)_n-$, or -NR-;
R is methyl or ethyl;
$R_1$ is hydrogen or halo;
$R_2$ is halo or trifluoromethyl;
n is 0-2;
provided that:
B is a group other than $-CH_2-$only when A and D are both $-CH_2CH_2-$or $-CH_2-$; and
D is $-CH_2S(O)_n-$only when A is -O-, $-S(O)_n-$, -N(ethyl)-or $-CH_2-$;
no more than two of A, B and D are $-CH_2-$.

This invention also provides a method of treating susceptible neoplasms in mammals which comprises administering to a mammal in need of such treatment an effective amount of a compound of this invention.

In addition, this invention provides pharmaceutical formulations comprising a compound of formula I in combination with a suitable pharmaceutical carrier, diluent, or excipient. These formulations are particularly useful in treating mammals suffering from susceptible neoplasms.

The term "halo" refers to fluoro, chloro, bromo, and iodo. The term "$C_1$-$C_3$ alkyl" refers to methyl, ethyl, propyl, and isopropyl.

The compounds of formula I are generally referred to as derivatives of N-([(substituted phenyl)amino]-carbonyl)arylsulfonamides. Alternatively, the compounds are referred to as 1-(substituted phenyl)-3-(arylsulfonyl)ureas.

The compounds of formula I may be prepared by a number of methods known in the literature.

A preferred method of preparing the compounds of formula I is that of the reaction of a sulfonylisocyanate of the formula II

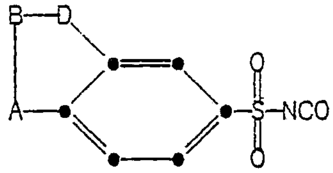

II

with an aniline derivative of the formula III

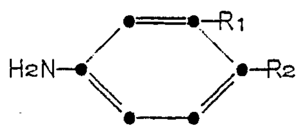

III

where A, B, D, $R_1$, and $R_2$ are the same as previously defined.

The reaction between compounds II and III is usually performed using equimolar amounts of the two reactants, although other ratios are operative. The reaction is best carried out in an aprotic non-reactive solvent such as benzene, toluene, acetonitrile, diethyl ether, tetrahydrofuran, dioxane, or preferably methylene chloride. The reaction may be carried out at temperatures from about 0° C up to the boiling point of the reaction mixture. At the preferred temperature range of about 20-30° C, the reaction produces a strong exotherm and the reaction is usually complete within 1 hour. The product thus obtained is recovered by filtration and may be purified, if desired, by any number of methods known to those skilled in the art, such as chromatography or crystallization.

Alternatively, an appropriately substituted sulfonamide IV

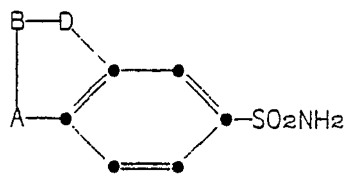

IV

may be reacted with an isocyanate of the formula V

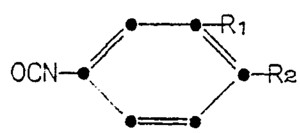

V

to provide the compounds of formula I. The reaction is generally carried out in a water miscible, non-reactive solvent such as tetrahydrofuran or acetone. Generally, an equimolar amount or slight molar excess of V is employed, although other ratios are operative. In addition, an aqueous solution of a base, such as sodium or potassium hydroxide, is employed. Usually the amount of base used is approximately equimolar to the amount of IV. The reaction is generally carried out from about 0° C up to the boiling point of the reaction mixture. At the preferred temperature range of 20-30° C, the reaction is usually complete within about three days.

The preferred method of preparing compounds of Formula I involves the reaction of sulfonamide IV with an alkyl haloformate to provide carbamate VI which is then reacted with aniline III to provide the corresponding product I

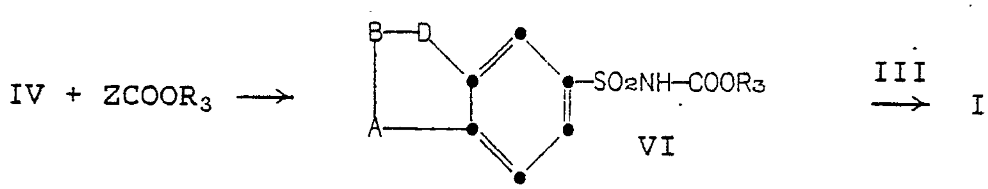


where Z is bromo or chloro and $R_3$ is $C_1$-$C_3$ alkyl. The transformation of IV into VI is usually accomplished in a non-reactive solvent, such as acetone or methyl ethyl ketone, in the presence of an acid scavenger, such as an alkali metal carbonate, for example potassium carbonate. A molar excess of the haloformate is usually added, although other ratios are operative, and the reaction mixture heated at a temperature from about 30° C up to the reflux temperature for a period of 1-6 hours to provide the desired intermediate VI. Intermediate carbamate VI and aniline III are then heated together in an inert high-boiling solvent, such as dioxane, toluene, or diglyme, at temperatures from about 50° C up to the reflux temperature of the mixture to provide the desired product I.

Thus, a further aspect of the present invention provides a process for preparing a compound of formula

I, as defined above, which comprises reacting with a compound of the formula

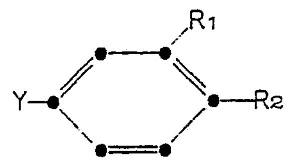

wherein Y is $-NH_2$ or -NCO, and $R_1$ and $R_2$ are as defined in Claim 1, a sulfonyl compound of the formula

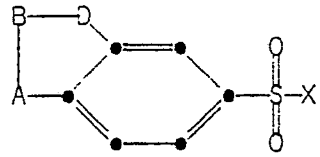

wherein X is -NCO, $-NH_2$ or $-NHCOOR_3$, wherein $R_3$ is $C_1$-$C_3$ alkyl, and A, B and D are as defined in claim 1, provided that if X is -NCO or $-NHCOOR_3$ then Y is $-NH_2$, and if X is $-NH_2$ then Y is -NCO.

Intermediates II, III, IV, and V and any other reagents required for other methods of preparation, are either commercially available, are known in the literature, or can be prepared by methods known in the art.

Certain intermediates of Formula IV, especially those wherein A and D are independently $-S(O)_n-$, $-CH_2S(O)_n-$, -O-or $-CH_2-$, may be prepared by chlorosulfonating an appropriately substituted benzene compound at 50°-130° C with a Villsmeier reagent prepared from sulfuryl chloride and dimethylformamide followed by ammonolysis with ammonia or ammonium hydroxide.

Intermediates wherein A or D are -SO-, $-SO_2-$, $-CH_2SO$-or $-CH_2SO_2$-are prepared by oxidizing the sulfur atoms with a conventional oxidizing agent, such as perbenzoic acid. Compounds wherein both A and D are sulfur-containing moieties, and are in different oxidation states, are prepared by carrying out the oxidation under mild conditions and separating the resulting mixture of products. For example, the oxidation may be carried out at low temperatures and in the presence of low concentrations of oxidizing agent to achieve mild oxidation conditions.

The following examples further illustrate the preparation of the compounds of this invention. The examples are illustrative only and are not intended to limit the scope of the invention in any way.

Example 1

N-([(4-chlorophenyl)amino]carbonyl)-1,3-dihydro-2-benzothiophene-5-sulfonamide

A 826 mg portion of 1,3-dihydro-2-benzothiophene-5-sulfonamide was dissolved in 3.8 ml of acetone, and 3.8 ml of 1N sodium hydroxide solution was added at ambient temperature. Then 591 mg of 4-chlorophenylisocyanate, dissolved in 3.8 mg of acetone, was added in one minute, with good stirring. The mixture was stirred for 19 minutes more, and then 3.8 ml of 1N hydrochloric acid was added and the mixture was stirred for 20 minutes more. The acetone was then removed under vacuum, and was replaced with 20 ml of ethyl acetate, which was also removed under vacuum. Thirty ml of ethyl acetate was added, and then the organic phase was separated and washed with 10 ml of brine. The organic phase was dripped through sodium sulfate, and was evaporated under vacuum to produce a foam. The residual foam was triturated with 10 ml of methylene chloride, and the solids remaining were collected, washed with more methylene chloride, and dried under vacuum to obtain 0.97 g of product, which was indicated by thin layer chromatography to be substantially pure. The elemental analysis was as follows.

Theory: C, 48.84; H, 3.55; N, 7.59; S, 17.39
Found: C, 49.00; H, 3.75; N, 7.68; S, 17.28

Example 2

N-([(4-chlorophenyl)amino]carbonyl)-2,3-dihydro-1-benzothiophene-5-sulfonamide

A 1.065 g portion of 2,3-dihydro-1-benzothiophene-5-sulfonamide was dissolved in 5 ml of acetone and was reacted with 775 mg of 4-chlorophenylisocyanate, substantially as shown in Example 1. The product was washed with water and dried under vacuum at 65° C to obtain 1.64 g of product, the elemental analysis of which was as follows.

Theory: C, 48.84; H, 3.55; N, 7.59; S, 17.39
Found: C, 49.12; H, 3.75; N, 7.52; S, 17.19

Example 3

N-([(4-chlorophenyl)amino]carbonyl)-2,3-dihydro-N-ethyl-1-benzopyrrole-5-sulfonamide

A 705 mg portion of 2,3-dihydro-N-ethyl-1-benzopyrrole-5-sulfonamide was dissolved in 3 ml of acetone, and was reacted with 479 mg of 4-chlorophenylisocyanate as described in Example 1. The crude product was taken up in 10 ml of methylene chloride and 10 ml of toluene, and the volume was slowly reduced to 5 ml. A small amount of hexane was then added, and the mixture was scratched and subjected to flash evaporation to begin precipitation. After the mixture was allowed to stand at ambient temperature, the solid product was collected, washed with toluene, and dried under vacuum at 65° C to obtain 1.02 g of the desired product, in slightly impure form. Nuclear magnetic resonance analysis showed that it was the desired compound, but that some impurities were present.

The compounds of formula I have been shown to be active against transplanted mouse tumors in vivo.

To demonstrate the anti-tumor activity of the compounds of Formula I, the compounds were tested in animals bearing a 6C3HED lymphosarcoma, also known as the Gardner lymphosarcoma (GLS). Table 1 gives the results of several experiments in mice bearing this tumor when compounds were administered orally. In the Table, column 1 gives the example number of the compound; column 2, the dose level; and column 3, the percent inhibition of tumor growth. The results are the average of 10 animals per group as compared with a suitable control group.

## Table 1

Activity of the Compounds of Formula I Against the 6C3HED Lymphosarcoma*

| Compound of Example No. | Dose** | Percent Inhibition |
|---|---|---|
| 1 | 150 | 35 |
| | 300 | 91 |
| 2 | 150 | 32 |
| | 300 | 57 |
| 3 | 150 | 15 |
| | 300 | 48 |

*Tested in C3H mice.

**mg/kg administered orally in emulphor. Dosing began the day following inoculation. Compounds were dosed once every day for eight days.

The compounds of Formula I are antineoplastic agents and the invention provides a method of treating susceptible neoplasms. The method comprises administering a compound by various routes including the oral, rectal, transdermal, subcutaneous, intravenous, intramuscular, or intranasal routes, being usually employed in the form of a pharmaceutical composition. It is a special feature of these compounds that they are effective following oral administration. Such compositions are prepared in a manner well known in the pharmaceutical art and comprise at least one active compound. Accordingly, in addition to the novel compounds of Formula I, the invention also includes pharmaceutical compositions comprising as active ingredient a compound of Formula I associated with a pharmaceutically acceptable carrier.

In making the compositions of the present invention, the active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions and sterile packaged powders.

Some examples of suitable carriers, excipients, and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methyl-and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient by employing procedures well known in the art.

The compositions are preferably formulated in a unit dosage form, each dosage containing from about 5 to about 500 mg, more usually about 25 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

The active compounds are effective over a wide dosage range. For example, dosages per day will normally fall within the range of about 0.5 to about 600 mg/kg of body weight. In the treatment of adult humans, the range of about 1 to about 50 mg/kg, in single or divided doses, is preferred. However, it will be

understood that the amount of the compound actually administered will be determined by a physician, in the light of the relevant circumstances including the condition to be treated, the choice of compound to be administered, the chosen route of administration, the age, weight, and response of the individual patient, and the severity of the patient's symptoms, and therefore the above dosage ranges are not intended to limit the scope of the invention in any way.

The following formulation examples may employ as active compounds any of the compounds of Formula I. The examples are illustrative only and are not intended to limit the scope of the invention in any way.

Example 4

Hard gelatin capsules are prepared using the following ingredients:

| | Quantity (mg/capsule) |
|---|---|
| N-([(4-chlorophenyl)amino]-carbonyl)-1,3-dihydro-2-benzo-thiophene-5-sulfonamide | 250 |
| Starch dried | 200 |
| Magnesium stearate | 10 |

The above ingredients are mixed and filled into hard gelatin capsules in 460 mg quantities.

Example 5

A tablet formula is prepared using the ingredients below:

| | Quantity (mg/tablet) |
|---|---|
| N-([(4-chlorophenyl)amino]-carbonyl)-1,3-dihydro-2-benzo-thiophene-5-sulfonamide | 250 |
| Cellulose, microcrystalline | 400 |
| Silicon dioxide, fumed | 10 |
| Stearic acid | 5 |

The components are blended and compressed to form tablets each weighing 665 mg.

Example 6

Tablets each containing 60 mg of active ingredient are made up as follows:

| | |
|---|---|
| N-([(4-chlorophenyl)-amino]carbonyl)2,3-dihydro-1-benzothiophene-5-sulfonyl | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60 °C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Example 7

Capsules each containing 80 mg of medicament are made as follows:

| | |
|---|---|
| N-([(4-chlorophenyl)amino]-carbonyl)-2,3-dihydro-N-ethyl-1-benzopyrrole-5-sulfonamide | 80 mg |
| Starch | 59 mg |
| Microcrystalline cellulose | 59 mg |
| Magnesium stearate | 2 mg |
| Total | 200 mg |

The active ingredient, cellulose, starch and magnesium stearate are blended, passed through a No. 45 mesh U.S. sieve, and filled into hard gelatin capsules in 200 mg quantities.

Example 8

A suspension containing 50 mg of medicament per 5 ml dose is made as follows: N-([(4-chlorophenyl)-amino]carbonyl)-2,3-dihydro-N-ethyl-1-benzopyrrole-5-sulfonamide 50 mg
Sodium carboxymethyl cellulose 50 mg
Syrup 1.25 ml
Benzoic acid solution 0.10 ml
Flavor q.v.
Color q.v.

Purified water to 5 ml

The medicament is passed through a No. 45 mesh U.S. sieve and mixed with the sodium carboxymethyl cellulose and syrup to form a smooth paste. The benzoic acid solution, flavor and color are diluted with some of the water and added, with stirring. Sufficient water is then added to produce the required volume.

Example 9

Capsules each containing 150 mg of medicament are made as follows:

| | |
|---|---|
| N-([(4-chlorophenyl)amino]carbonyl)-2,3-dihydro-1-benzothiophene-5-sulfonamide | 150 mg |
| Starch | 164 mg |
| Microcrystalline cellulose | 164 mg |
| Magnesium stearate | 22 mg |
| Total | 500 mg |

The active ingredient, cellulose, starch and magnesium stearate are blended, passed through a No. 45 mesh U.S. sieve, and filled into hard gelatin capsules in 500 mg quantities.

Example 10

Tablets containing 80 mg of medicament each are made as follows:

| | |
|---|---|
| N-([(4-chlorophenyl)amino]-carbonyl)-1,3-dihydro-2-benzothiophene-5-sulfonamide | 80 mg |
| Starch | 40 mg |
| Microcrystalline cellulose | 40 mg |
| Polyvinylpyrrolidone (as 10% aqueous solution) | 5 mg |
| Magnesium Stearate | 1 mg |
| Talc | 2 mg |
| Total | 168 mg |

Process as described in Example 6 and compress into tablets weighing 168 mg each.

Example 11

Capsules containing 100 mg each of medicament are made as follows:

| | |
|---|---|
| N-([(4-chlorophenyl)amino]-carbonyl)-2,3-dihydro-1-benzo-thiophene-5-sulfonamide | 100 mg |
| Starch | 60 mg |
| Magnesium stearate | 5 mg |
| Microcrystalline cellulose | 60 mg |
| Total | 225 mg |

The ingredients are blended, passed through a sieve, and filled into hard gelatin capsules in 200 mg quantities.

Example 12

Capsules containing 125 mg of medicament each are made as follows:

| | |
|---|---|
| N-([(4-chlorophenyl)amino]-carbonyl)-1,3-dihydro-2-benzo-thiophene-5-sulfonamide | 125 mg |
| Starch | 50 mg |
| Microcrystalline cellulose | 70 mg |
| Talc | 5 mg |
| Total | 250 mg |

Process as described in Example 7 and fill capsules containing 250 mg each.

Example 13

Capsules containing 90 mg of medicament each are made as follows:

| | |
|---|---|
| N-([(4-chlorophenyl)amino]-carbonyl)-2,3-dihydro-1-benzo-thiophene-5-sulfonamide | 90 mg |
| Starch | 55 mg |
| Microcrystalline cellulose | 52 mg |
| Talc | 3 mg |
| Total | 200 mg |

Process as described in Example 7 and fill in 200 mg quantities.

## Claims

1. A compound of the formula

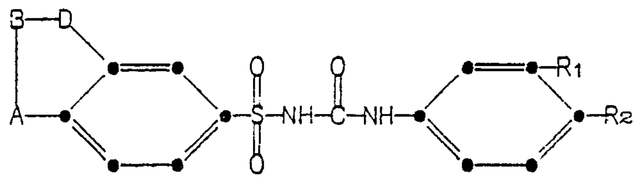

I

wherein A is $-S(O)_n-$, $-CH_2S(O)_n-$, -N(ethyl)-, or $-CH_2-$;
D is $-CH_2-$, $-S(O)_n-$, -NR-, or $-CH_2S(O)_n-$;
B is $-CH_2-$, -O-, $-S(O)_n-$, or -NR-;
R is methyl or ethyl;
$R_1$ is hydrogen or halo;
$R_2$ is halo or trifluoromethyl;
n is 0-2;
provided that:
B is a group other than $-CH_2-$only when A and D are both $-CH_2CH_2-$or $-CH_2-$; and
D is $-CH_2S(O)_n$ only when A is -O-, $-S(O)_n-$, -N(ethyl)-or $-CH_2-$;
no more than two of A, B and D are $-CH_2-$.

2. A compound according to claim 1 wherein $R_1$ is hydrogen.

3. A compound according to claim 2 wherein $R_2$ is halo.

4. N-{[(4-Chlorophenyl)amino]carbonyl}-1,3-dihydro-2-benzothiophene-5-sulfonamide.

5. N-{[(4-Chlorophenyl)amino]carbonyl}-2,3-dihydro-1-benzophene-5-sulfonamide.

6. N-{[(4-Chlorophenyl)amino]carbonyl}-2,3-dihydro-N-ethyl-1-benzopyrrole-5-sulfonamide.

7. A compound of any one of claims 1 to 6 for use as an antineoplastic agent.

8. A pharmaceutical formulation comprising as the active ingredient a compound of any one of claims 1 to 6 associated with one or more pharmaceutically-acceptable carriers or excipients therefor.

9. A process for preparing a compound of formula I, as defined in any one of claims 1-6, which comprises reacting with a compound of the formula

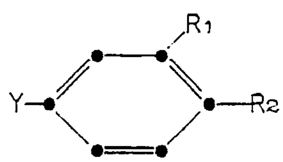

wherein Y is $-NH_2$ or -NCO, and $R_1$ and $R_2$ are as defined in claim 1, a sulfonyl compound of the formula

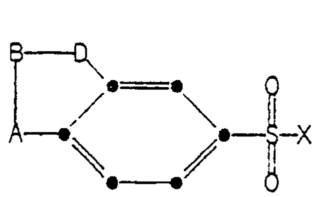

wherein X is -NCO, $-NH_2$ or $-NHCOOR_3$, wherein $R_3$ is $C_1$-$C_3$ alkyl, and A, B and D are as defined in claim 1, provided that if X is -NCO or $-NHCOOR_3$ then Y is $-NH_2$, and if X is $-NH_2$ then Y is -NCO.

Claims for the following Contracting States : ES, GR

1. A process for preparing a compound of the formula

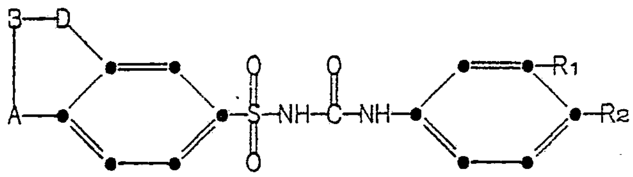

I

wherein
A is $-S(O)_n-$, $-CH_2S(O)_n-$, -N(ethyl)-, or $-CH_2-$;
D is $-CH_2-$, $S(O)_n-$, -NR-, or $-CH_2S(O)_n-$;
B is $-CH_2-$, -O-, $-S(O)_n-$, or -NR-;
R is methyl or ethyl;
$R_1$ is hydrogen or halo;
$R_2$ is halo or trifluoromethyl;
n is 0-2;
provided that:
B is a group other than $-CH_2-$only when A and D are both $-CH_2CH_2-$or $-CH_2-$; and
D is $-CH_2S(O)_n$ only when A is -O-, $-S(O)_n-$, -N(ethyl)-or $-CH_2-$;
no more than two of A, B and D are $-CH_2-$; which comprises reacting with a compound of the formula

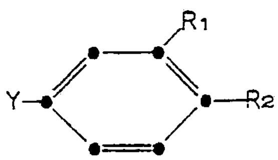

wherein Y is $-NH_2$ or -NCO, and $R_1$ and $R_2$ are as defined in claim 1, a sulfonyl compound of the formula

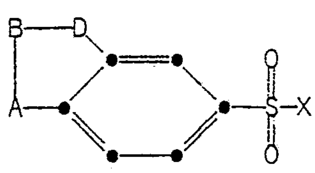

wherein X is -NCO, $-NH_2$ or $-NHCOOR_3$, wherein $R_3$ is $C_1$-$C_3$ alkyl, and A, B and D are as defined in claim 1, provided that if X is -NCO or $-NHCOOR_3$ then Y is $-NH_2$, and if X is $-NH_2$ then Y is -NCO.

2. A process of claim 1 for preparing a compound wherein $R_1$ is hydrogen.

3. A process of claim 2 for preparing a compound wherein $R_2$ is halo.

4. A process of any one of claims 1-3 for preparing N-{[(4-chlorophenyl)amino]carbonyl}-1,3-dihydro-2-benzothiophene-5-sulfonamide.

5. A process of any one of claims 1-3 for preparing N-{[(4-chlorophenyl)amino]carbonyl}-2,3-dihydro-1-benzothiophene-5-sulfonamide.

6. A process of any one of claims 1-3 for preparing N-{[(4-chlorophenyl)amino]carbonyl}-2,3-dihydro-N-ethyl-1-benzopyrrole-5-sulfonamide.

7. A process for preparing a pharmaceutical formulation comprising admixing a compound of formula I as defined in claim 1 with one or more pharmaceutically-acceptable carriers or excipients therefor.